# EUROPEAN PATENT APPLICATION

(11) **EP 0 917 878 A1**
(43) Date of publication of application: **26.05.1999**
(21) Application number: 98905677.5
(22) Date of filing: 26.02.1998
(51) Int. Cl.: A61K 45/00, A61K 31/70, A61K 38/17, A61K 39/395

(54) **REMEDIES FOR HEPATITIS**

(30) Priority: 26.02.1997 JP 42493/97
(71) Applicant: TORAY INDUSTRIES, INC., Tokyo 103-8666 (JP)
(72) Inventor: TANAKA, Toshiaki, Zushi-shi, Kanagawa 249-0004 (JP); MORIYA, Keiko, Yokohama-shi, Kanagawa 232-0064 (JP); KAINOH, Mie, Fujisawa-shi, Kanagawa 251-0052 (JP)
(74) Representative: Kador & Partner
(86) International application number: JP9800802
(87) International publication number: WO9837914

(57) **Abstract**

The present invention relates to a therapeutic agent for hepatitis, comprising an integrin inhibitor as an active ingredient. The integrin inhibitors which can be used here include antibodies, proteins other than antibodies, polypeptides, peptides, nucleic acids, saccharides, their derivatives, low molecular weight compounds, etc. respectively capable of being bound to an integrin. In the present invention, especially anti-VLA-4 antibodies, VLA-4 inhibiting peptides and VLA-4 inhibiting low molecular weight compounds are effective as therapeutic agents for hepatitis.

## Description

### Technical Field

The present invention relates to the therapy of hepatitis. In more detail, it relates to the application of a substance inhibiting the cell adhesion through an integrin, particularly VLA-4 molecule acting as an adhesion molecule onto the cell surface, as a therapeutic agent for hepatitis.

### Background Arts

Various cells have receptors which mediate the adhesion between cells and receptors which mediate the adhesion between cells and extracellular matrices, and these receptors play important roles in immune reaction, inflammatory reaction, development, morphogenesis, wound healing, hemostasis, cancerous metastasis, etc. By isolating and identifying the receptors which participate in these phenomena, the existence of so-called cell adhesion molecules has been clarified. Many of the molecules identified one after another are classified in reference to their structural features into integrin superfamily, immunoglobulin superfamily, selectin family, cadherin family, etc. (Corlos, T. M. and Harlan, J. M., Blood, 84, 2068-2101 (1994), Albelda, S. M. and Buck, C. A. FASEB I. 2868-2880 (1990)).

A receptor belonging to the integrin superfamily has a heterodimeric structure in which two subunits, α-chain and β-chain as mutually different membrane proteins are associated with each other non-covalently (Hynes, R. O., Cell, 48, 549-554 (1987)). In the past, the molecules belonging to the integrin superfamily were classified into three subfamilies; β1 integrin, β2 integrin and β3 integrin. Later, new β chains and α chains were discovered one after another, and presently eight β chains (β1, β2, β3, β4, β5, β6, β7 and β8) and fifteen α chains (α1, α2, α3, α4, α5, α6, α7, α8, α9, αv, αL, αM, αX, α 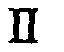 b and αE) have been identified (Corlos, T. M. and Harlan, J. M. Blood 84, 2068-2101 (1994), Elner, S. G. and Elner, V. M. Inv. Ophtal. Vis. Sci., 37, 696-701 (1996)). Twenty one integrin molecules in which an α chain and a β chain are associated with each other have been identified so far (Corlos, T. M. and Harlan, J. M. Blood 84, 2068-2101 (1994), Elner, S. G. and Elner, V. M., Inv. Ophtal, Vis. Sci., 37, 696-701 (1996)). In the β1 integrin family of the three subfamilies classified in reference to β chains, ten molecules of α1β1, α2β1, α3β1, α4β1, α5β1, α6β1, α7β1, α8β1, α9β1 and αvβ1 have been identified. Among them, nine molecules excluding αvβ1 are called VLAs, since they were identified as antigens expressed on cell surfaces in the later stage of lymphocyte activation, i.e., as very late activation antigens, and they are called VLA-1 ∼ VLA-9 respectively in reference to the α chains.

It has been already attempted to inhibit the cell adhesion through these integrin molecules, for treatment of diseases (Drug and Market Development 6, 201-205 (1995), Drugs of the Future 21, 283-289 (1996)). For example, integrin α4β1 (also called VLA-4) inhibitors are being developed as therapeutic agents for multiple screlosis and inflammatory diseases, and integrin αLβ2 (also called LFA-1) and integrin αMβ2 (also called Mac-1) inhibitors are being developed as therapeutic agents for inflammatory diseases, while integrin αvβ3 inhibitors are being developed as vascularization inhibitors, cancerous metastasis preventives and osteoporosis therapeutic agents (Drug and Market Development 6, 201-205 (1995), Drugs of the Future 21, 283-389 (1996)). An integrin α 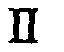 bβ3 (also called GPIIb/IIIa) blocking antibody is already marketed as an anti-platelet agent (Drugs of the Future 21, 283-389 (1996)).

Experiments using animal models suggest that said integrin inhibitors can be used for treating other diseases (Clinical Immunology and Immunopathology 80, S15-S22 (1996)). Especially, it is indicated that especially VLA-4 inhibitors can be therapeutic agents for multiple screlosis, inflammatory colitis, allergic asthma, diabetes, contact hypersensitivity, nephritis and organ transplant rejection (Drugs of the Future 21, 283-289 (1996), J. Clin. Invest. 94, 1722-1728 (1994), Clinical Immunology and Immuno-pathology 80, S15-S22 (1996)).

As described above, the possibility that integrin inhibitors can be therapeutic agents for various diseases is indicated. However, the possibility as a therapeutic agent for hepatitis has not been studied.

### Brief Description of the Drawings

Fig. 1 shows that α4·IgG heavy chain-β1·IgG heavy chain chimeric protein heterodimer complex is bound to CS-1 peptide, and that the binding is inhibited by an anti-integrin antibody or EDTA used as a cation chelating agent.
Fig. 2 shows that the binding between α4·IgG heavy chain-β1·IgG heavy chain chimeric protein heterodimer complex and CS-1 peptide is inhibited by GPEILDVPST, and is not inhibited by other peptides.

### Disclosure of the Invention

The object of the present invention is to provide a new therapeutic agent for hepatitis.

The inventors studied intensively and as a result, found that a substance inhibiting the cell adhesion through an adhesion molecule integrin on the cell surface can be used as a therapeutic agent for hepatitis. Thus, the present invention has been completed.

The present invention has the following technical features:
[1] A therapeutic agent for hepatitis, comprising an integrin inhibitor as an active ingredient.
[2] A therapeutic agent for hepatitis, according to said [1], wherein the integrin inhibitor is at least one selected from a group consisting of antibodies, proteins other than antibodies, polypeptides, peptides, nucleic acids, saccharides, their derivatives and low molecular weight compounds, respectively capable of being bound to an integrin.
[3] A therapeutic agent for hepatitis, according to said [1] or [2], wherein the integrin is an integrin comprising one α chain selected from α1, α2, α3, α4, α5, α6, α7, α8, α9, αv, αL, αM, αX, αIIb and αE, and one β chain selected from β1, β2, β3, β4, β5, β6, β7 and β8.
[4] A therapeutic agent for hepatitis, according to said [1] or [2], wherein the integrin is a VLA.
[5] A therapeutic agent for hepatitis, according to said [1] or [2], wherein the integrin is an integrin with α4 as the α chain.
[6] A therapeutic agent for hepatitis, according to said [4] wherein the VLA is a VLA-4.
[7] A therapeutic method for hepatitis, comprising the step of administering an integrin inhibitor to a patient of hepatitis.
[8] A therapeutic method for hepatitis, according to said [7], wherein the integrin inhibitor is at least one selected from a group consisting of antibodies, proteins other than antibodies, polypeptides, peptides, nucleic acids, saccharides, their derivatives and low molecular weight compounds, respectively capable of being bound to an integrin.
[9] A therapeutic method for hepatitis, according to said [7] or [8], wherein the integrin is an integrin comprising one α chain selected from α1, α2, α3, α4, α5, α6, α7, α8, α9, αv, αL, αM, αX, α 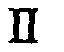 b and αE ,and one β chain selected from β1, β2, β3, β4, β5, β6, β7 and β8.
[10] A therapeutic method for hepatitis, according to said [7] or [8], wherein the integrin is a VLA.
[11] A therapeutic method for hepatitis, according to said [7] or [8], wherein the integrin is an integrin with α4 as the α chain.
[12] A therapeutic method for hepatitis, according to said [10] wherein the VLA is a VLA-4.

### The Best Embodiments of the Invention

The integrin inhibitor in the present invention refers to a substance inhibiting the cell adhesion through a molecule belonging to an integrin superfamily. The integrin superfamily refers to a series of molecules having a heterodimeric structure in which two membrane proteins of an α chain and a β chain are associated with each other non-covalently, and each of the integrins is a pair in which an α chain and a β chain are associated with each other. Twenty one molecules obtained by combining fifteen a chains of α1, α2, α3, α4, α5, α6, α7, α8, α9, αv, αL, αM, αX, α 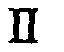 b and αE and eight β chains of β1, β2, β3, β4, β5, β6, β7 and β8 have been identified so far (Corlos, T. M. and Harlan, J. M. Blood 84, 2068-2101 (1994), Elner, S. G. and Elner, V. M. Inv. Ophtal. Vis. Sci., 37, 696-701 (1996)).

The integrins which can be preferably used in the present invention include integrins respectively consisting of one α chain selected from α1, α2, α3, α4, α5, α6, α7, α8, α9, αv, αL, αM, αX, α 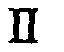 b and αE and one β chain selected from β1, β2, β3, β4, β5, β6, β7 and β8. Among them, those belonging to integrin β1 family in which one of nine α chains (α1, α2, α3, α4, α5, α6, α7, α8, α9) and β1 are combined, called VLAs are preferable, and it is more preferable that the α chain is α4. Especially integrin α4β1 called VLA-4 is preferable.

Substances which can be used to inhibit the above integrins include those which can be bound to the integrins, for example, antibodies, proteins other than antibodies, polypeptides, peptides, nucleic acids, saccharides, their derivatives and low molecular weight compounds, etc.

Among the antibodies, anti-integrin antibodies with cell adhesion inhibitory action are preferable. Furthermore, anti-VLA antibodies which inhibit the adhesion through any of the integrins called VLAs are preferable, and it is especially preferable to use an anti-VLA-4 antibody. In addition to intact molecules of these antibodies, their parts such as Fab, Fab', F(ab')2, F(v) fragment, and a peptide formed by VLA-4 binding epitope of an antibody can also be used. A humanized chimeric antibody can also be used. Methods for monoclonal antibody preparation are known as general experimental techniques and described in detail in various published books (e.g., "Antibody" ed. Harlow, E. and Lane, D. (1988), Cold Spring Harbor Laboratory, NY). An anti-integrin antibody can be prepared according to any of the methods described in these books. In detail, a purified integrin protein or cells expressing an integrin are injected into a mouse or rat, etc. for immunization, and spleen cells of the immunized animal and myelomas are fused together to form hybridomas. Hybridomas capable of producing an anti-integrin antibody can be selected by confirming that the cultured supernatant solution of the hybridomas reacts with a protein or cells used as an immunogene or other cells expressing the intended integrin, and inhibits the cell adhesion through the intended integrin. It can be confirmed by immunoprecipitation that the antibody produced by the selected hybridomas recognizes the intended integrin molecule. If the obtained hybridomas are cultured according to an ordinary cell culture method, antibodies can be produced in the culture supernatant. As another method, if they are inoculated into peritoneal cavity, the antibody can be produced in the ascites fluid. Furthermore, such an antibody-containing fluid can be treated by a known method for purifying the antibody.

Moreover, a known antibody for inhibiting the cell adhesion through integrin, especially VLA-4 molecule can be used. For example, as murine anti-VLA-4 antibodies, PS/2 (Miyake, K. J. Exp. Med. 113, 599-607 (1991)) and R1-2 (Holzman, B. et al., Cell 56, 37-46 (1989)), etc. are known. Since hybridomas capable of producing these antibodies can be obtained from ATCC (PS/2: CRL1911, R1-2: HB227), these hybridomas can be cultured to prepare the antibodies. Marketed purified antibodies can also be used (PS/2: Southern Biotechnology, R1-2: Fujisawa Pharmaceutical Co., Ltd.). Furthermore, hybridomas capable of producing an antibody for inhibiting the cell adhesion through an integrin can be newly obtained by said method, to prepare an antibody. Several anti-humanVLA-4 antibodies are known (Sanchez-Madrid, F. et al., Eur. J. Immunol. 16, 1343-1349 (1986), Hemler, M. E. et al., J. Biol. Chem. 262, 11478-11485 (1987). Clayberger, C. et al., J. Immunol. 138, 1510-1514 (1987)), and they can be prepared according to the methods described in the respective reports. Marketed purified antibodies (HP2.1: Coal tar K.K., L25: Nippon Becton Dickinson K.K.) can also be used. The antibodies in the present invention are not limited to those described so far, and any monoclonal antibodies with activity to inhibit the cell adhesion through an integrin are included in the integrin inhibitors referred to in the present invention.

The substances capable of being bound to an integrin other than antibodies, i.e., proteins, polypeptides, peptides, nucleic acids, saccharides, their derivatives and low molecular weight compounds must be integrin inhibitors with inhibitory action on cell adehesion. Furthermore, those inhibiting the adhesion through any of integrins called VLAs and those inhibiting the adhesion through an integrin with α4 chain are preferable. Especially a VLA-4 inhibitor is preferable. These inhibitors include fragments of ligands of integrins and their mutants. As a fragment of a ligand, it is preferable to use that containing an epitope contributing for binding to an integrin. For example, a fragment obtained by digesting a ligand protein by protease treatment (e.g., Ruoslahti, E. et al., J. Biol. Chem. 256, 7277-7281 (1981)), a fragment of a ligand obtained by peptide synthesis (e.g., Humphries, M. J. et al., J. Biol. Chem. 262, 6886-6892 (1987)), or a fragment expressed as a protein mutant (e.g., Mould, A. P. et al., J. Biol. Chem. 269, 27224-27230 (1994)), etc. can be used. For example, a peptide containing CS-1 region which is a VLA-4 binding epitope of fibronectin can be used.

However, the fragments of ligands are not limited to the CS-1 peptide.

As possible sources of an inhibitor, proteins, polypeptides, peptides, nucleic acids, saccharides, low molecular weight compounds and their derivatives can be purchased or prepared, to select a substance inhibiting the binding between an integrin and a ligand from them. For selection, an integrin protein or integrin expressing cells and a test sample are mixed beforehand, and the mixture is caused to react with a ligand protein or ligand-expressing cells for an appropriate time, to evaluate the affinity of the integrin to the ligand. If the affinity declines, the test sample can be judged to be an inhibitor.

As another method, at first integrin binding substances can be selected, to look for an inhibitor among them. For example, as sources of binding substances, a phage peptide library (e.g., Scott, J. K. and Smith, G. P., Science 249, 386-390 (1990)) or a DNA oligomer library (e.g., O'Connel, D. et al., Proc. Natl. Acad. Sci. USA 93, 5883-5887 (1996)), etc. can be prepared, and caused to react with an integrin formed as a solid phase on a plate, for selecting binding substances (e.g., Healy, J. M. et al., Biochemistry 34, 3948-3955 (1995)), and an inhibitor can be looked for according to the above method.

For selecting a VLA-4 inhibitor, can be used a general adhesion evaluation system which measures the number of adhered-Jurkat cells expressing VLA-4 on the cell surfaces to a ligand formed as a solid phase. As shown in Examples 3 to 5, it is also possible to use a heterodimer complex (α4-immunoglobulin heavy chain-β1·immunoglobulin heavy chain chimeric protein heterodimer complex, i.e., a chimeric protein of VLA-4 and an immunoglobulin heavy chain) in which a chimeric molecule being composed with the extracellular region of integrin α4 and an immunoglobulin heavy chain (integrin α4· immunoglobulin heavy chain chimeric protein) and a chimeric molecule being composed with the extracellular region of integrin β1 and an immunoglobulin heavy chain (integrin β1· immunoglobulin heavy chain chimeric protein) are associated with each other. The immunoglobulin to be bound to VLA-4 can be the isotype of IgG, IgM or IgE or its subclass heavy chain or light chain. In the present invention, it is preferable to use IgG₁ heavy chain. For testing the inhibitory effect of a sample using α4·IgG heavy chain-β1·IgG heavy chain chimeric protein heterodimer complex, it is desirable to use the heterodimer complex and the sample as a mixture beforehand.

In the present invention, the effect of the integrin inhibitors obtained as described above is illustrated using a murine hepatitis model in Examples 1 and 2, but the integrin inhibitors manifest a similar effect also in the hepatitis of other animal species. To obtain the therapeutic effect in mice, it is desirable to use an antibody or any other inhibitors against a murine integrin, and to obtain the therapeutic effect in human or other animal species, it is desirable to use an antibody or any other inhibitors reacting with an integrin of human or other animal species.

In the present invention, the therapeutic effect of the integrin inhibitor for hepatitis was confirmed using a murine ConA induced hepatitis model. This model is considered to have a similar mechanism the T cell mediated hepatopathy among disease conditions of human hepatitis (Lohse, A. W. et al., P.191-199 in "Auto-immune disease models" ed. Cohen, I. R. and Miller, A. (1994), Academic Press, California, Tiegs, G. et al., J. Clin. Invest. 90, 196-203 (1992), Mizushima, H. et al., J. Exp. Med. 179, 1529-1537 (1994)). Therefore, it has been clarified that the integrin inhibitor of the present invention is useful for treating hepatitis of animals including human, above all, T cell mediating hepatopathy. The T cell mediating hepatopathy includes, for example, acute virus hepatitis, chronic virus hepatitis, drug hepatopathy, fulminating hepatitis, autoimmune hepatopathy, chronic hepatitis, etc.

The therapeutic agent containing the integrin inhibitor of the present invention as an active ingredient can contain a pharmaceutically allowable carrier (carrier, excipient or diluent). The formulations which can be used include a powder, granules, tablets, capsules, injection, suppositories, slow-releasing drug, etc. The therapeutic agent can be administered, for example, orally, nasotracheally, transpulmonarily, subcutaneously, intramuscularly, intravenously, intra-arterially and non-orally. A preferable dose is about 0.01 to 1000 mg/kg per time, but it depends on the integrin inhibitor used and the patient treated.

### Examples

The details of the present invention are described below in reference to examples, but the present invention is not limited thereto or thereby.

### [Example 1]

### Effect of an antibody inhibiting the cell adhesion through VLA-4 molecular, on hepatopathy:

Male 8-week-old BALB/c mice were administered with 150 µg/body of an anti-VLA-4 antibody (Clone PS/2) intraperitoneally, and 16 hours later, administered with concanavaline A (ConA) by 20 mg/kg intravenously, to induce hepatitis. Eight hours after administration of ConA, the peripheral blood was sampled and centrifuged, to obtain a serum. Aspartato aminotransferase (AST) and alanine aminotransferase (ALT) as liver function markers were determined using a biochemical analyzer (Ciba Corning 550), as indicators of hepatitis intensity. The results are shown in Table 1 as a mean value ± standard error respectively. The experiment was performed with six mice per group.

**Table 1**

| | AST (U/L) | ALT (U/L) |
|---|---|---|
| Normal group | 164.9±24.2 | 62.4±2.3 |
| ConA administered group | 2680.0±870.6 | 6197.5±2286.3 |
| Anti-VLA-4 antibody + ConA administered group | 715.8±180.5 | 1029.2±499.8 |

As a result, as shown in Table 1, the administration of ConA increased the hepatopathy indicators of AST and ALT, but they were obviously decreased by the anti-VLA-4 antibody. It had been confirmed that the administration of rat IgG2b (Purified Rat Myeloma IgG2b, ZYMED) of the same isotype as PS/2 did not affect the increase of AST or ALT. This result suggests that the inhibition of adhesion mediated by VLA-4 is effective for treating hepatopathy.

### [Example 2]

### Effect of a peptide capable of inhibiting the binding to VLA-4, on hepatopathy:

Male 8-week-old BALB/c mice were administered with 300µ g/body of a fibronectin partial peptide H/120 (peptide containing CS-1 and CS-5 as VLA-4 bound epitopes) prepared according to a reported method (Mould, A. P. et al., J. Biol. Chem. 269, 27224-27230 (1994)) twice intravenously , 16 hours before and at the time of administration of ConA, and administered with ConA by 20 mg/kg intravenously as described for Example 1, to induce hepatitis. Eight hours after administration of ConA, the peripheral blood was sampled and centrifuged to obtain a serum, and the AST and ALT in the serum were determined. The experiment was performed with 8 or 9 mice per group.

As a result the fibronetcin partial peptide H/120 inhibited the increase of AST by ConA administration by 15.4%, and the increase of ALT by 32.4%. This result suggests, as in Example 1, that a VLA-4 inhibitor is effective for treating hepatopathy.

### [Example 3]

### Preparation of α4·IgG heavy chain-β1·IgC heavy chain chimeric protein heterodimer complex (chimeric protein of VLA-4 and IgG heavy chain):

### (1) Construction of human IgG₁ heavy chain expression vector

As human IgG₁ genome gene, a clone identical with reported base sequence information (Ellison, J. W. et al., Nucleic Acids Res., 10, 4071-4079 (1982)) was acquired from a human genomic library (CLONTECH) using a hybridization cDNA probe based on the sequence information. This was used as the template DNA of PCR. As primers for amplifying the DNA fragment containing the hinge region (H) and the constant region portions (CH2 and CH3) of the human IgG₁ gene, a DNA oligomer shown in sequence No. 1 of the sequence table (hereinafter a sequence No. of the sequence table is simply called a sequence No.) with BamH I restriction site and a DNA oligomer shown in sequence No. 2 with Xba I restriction site were synthsized.
5' -GCGGATCCCGAGCTGCTGGAAGCAGGCTCAG-3' (Sequence No. 1)
5' -CCTCTAGACGGCCGTCGCACTCATTTA-3' (Sequence No. 2)

The template DNA, primers, dNTPs (an equimolar mixture of dATP, dCTP, dGTP and dTTT) and Taq polymerase (Takara) were mixed in a PCR buffer (100 mM Tris-HCl, 500 mM KCl, 15 mM MgCl₂, 0.01% gelatin, pH 8.3), and in a thermal cycler (Perkin Elmer Cetus), the mixture was treated at 94°C for 1 minute for DNA denaturation, at 58°C for 2 minutes for annealing the primers and at 72°C for 3 minutes for elongating the primers. This treatment was performed 30 cycles. The amplified DNA was digested by restriction enzymes BamH I and Xba I, and the DNA fragment was purified by 1% agarose gel according to a general method ("Antibody", Harlow, E. and Lane, D. (1988), Cold Spring Harbor Lab. Press, New York). It was linked, using a T4DNA ligase, with a large DNA fragment of pBluescriptSK(+) (STRATAGENE) purified and digested by restriction enzymes BamH I and Xba I. The plasmid DNA was used to transform Escherichia coli (JM109), and the transformant was selected, to obtain a plasmid DNA (IgG₁ Bluescript). Then, expression vector pcDL-SRα296 was digested by restriction enzyme BamH I, and blunted the termini by T4DNA polymerase treatment, and a Not I linker was linked. The large DNA fragment obtained by digesting it by restriction enzymes Not I and Xho I and the small DNA fragment obtained by digesting IgG₁ Bluescript by restriction enzymes Not I and Xho I were purified according to a general method, and linked by T4DNA ligase. It was transformed into Escherichia coli (HB101), and the transformant was selected, to obtain a plasmid DNA. Hereinafter this plasmid (IgG₁SRα) is called a human IgG₁ expression vector. In the following examples, since the basic protocol of gene manipulation is the same as above, the description will be simplified.

### (2) Construction of integrin α4·IgG heavy chain chimeric protein expression vector

The DNA fragment coding for the extracellular portion of integrin α4 was obtained with a design to use restriction sites EcoR I, Stu I and BamH I for linking based on reported cDNA sequence information (Takada, Y. et al., EMBO J., 8, 1361-1368 (1989)), by subcloning the region from the N terminus translation initiation site to Stu I restriction site as α4-1, the region from Stu I restriction site to EcoR I restriction site as α4-2, and the region from EcoR I restriction site to BamH I restriction site as α4-3, and linking the three regions. The detailed methods are described below.

The port ion coding for α4-1 was designed to be cloned by linking the DNA oligomers of sequence Nos. 3 to 6, and the DNA oligomers shown in sequence Nos. 3 to 6 were synthesized. For the sequence Nos. 3 and 4, restriction site Xba I was added on the side to code for the N terminus, for linking to a vector. Furthermore, by base substitution, restriction site Stu I was inserted on the side to code for the N terminus of sequence Nos. 5 and 6. The 5' termini of the synthesized oligomers were phosphated and annealed, and were linked using T4DNA ligase. After completion of linking, restriction enzymes Xba I and Stu I were used for cutting, and electrophoresis was effected by 5% agarose (NuSieve GTGagarose, FMC) gel. The intended DNA fragment (α4-1) of about 120 bp was cut out and purified.
5' -CTAGACCACCATGTTCCCCACCGAGAGCGCATGGCTTGGGAAGCGAGGCGCGAACCCGGGCCCCGGA GCTGCA-3' (Sequence No. 3)
5' -GCTTCGGGGCCCGGGTTCGCGCCTCGCTTCCCAAGCCATGCGCTCTCGTGGGGAACATGGTGGT-3' (Sequence No. 4)
5' -CTCCGGGAGACGGTGATGCTGTTGCTGTGCCTGGGGGTCCCGACCGGCAGG-3' (Sequence No. 5)
5' -CCTGCCGGTCGGGACCCCCAGGCACAGCAACAGCATCACCGTCTCCCGGAGTCGA-3 ' (Sequence No. 6)
   Then, the RNA of human osteosarcoma cell line MG63 (ATCC CRL 1427) as an integrin α4 expression cell was separated, and PolyA(+)RNA was purified using oligo dT cellulose column (NEB). Based on it, a single stranded cDNA was synthesized using a reverse transcriptase (GIBCO), and used as the template for PCR. As primers for amplifying α4-2 and α4-3 DNAs, four DNA oligomers of sequence Nos. 7 to 10 with Pst I and Stu I restriction sites inserted (sequence No. 7) or BamH I restriction site inserted (sequence No. 10) were synthesized.
5' -CACTGCAGGCAGGCCTTACAACGTGGACACTGAGAGC-3' (Sequence No. 7)
5' -GCAGAAACCTGTAAATCAGCAG-3' (Sequence No. 8)
5' -GCATTTATGCGGAAAGATGTGC-3' (Sequence No. 9)
5' -CGGGATCCGTGAAATAACGTTTGGGTCTT-3' (Sequence No. 10)

The template cDNA, primers, dNTPs and Taq polymerase were mixed in a PCR buffer, and the mixture was treated by a thermal cycler at 94°C for 1 minute for DNA denaturation, at 58°C for 2 minutes for annealing the primers and at 72°C for 3 minutes for elongating the primers. This treatment was performed 30 cycles. The amplified DNA fragments of α4-2 and α4-3 were digested by Pst I and EcoR I, or EcoR I respectively and BamH I and subcloned into pBluescriptKS(+) (STRATAGENE), to prepare plasmid DNAs (hereinafter called α4-2 Bluescript and α4-3 Bluescript). Then, upstream of the α4-2 Bluescript, α4-1 was linked using Xba I and Stu I restriction sites, to prepare a plasmid DNA (hereinafter called α4-1-2 Bluescript).

The α4-1-2 Bluescript was digested by restriction enzyme Not I, and blunted the termini by T4DNA polymerase treatment, being digested by restriction enzyme EcoR I, to prepare a small DNA fragment. The α4-3 Bluescript was digested by restriction enzymes EcoR I and BamH I, to prepare a small DNA fragment. The two small DNA fragments were simultaneously linked to a large DNA fragment obtained by digesting IgG₁SRα by restriction enzymes EcoR V and BamH I , to obtain a plasmid DNA. The plasmid (integrin α4·IgGSRα) is hereinafter called integrin α4·IgG heavy chain chimeric protein expression vector.

### (3) Construction of integrin β1·IgG heavy chain chimeric protein expression vector

The RNA of human fibroblast cell line MRC5 (ATCC CCL 171) as an integrin β1 expressing cell was separated, and oligo dT cellulose column was used to purify PolyA (+)RNA. Based on it, a single stranded cDNA was synthesized using a reverse transcriptase, and used as the template for PCR. As primers, two DNA oligomers of sequence Nos. 11 and 12 with BamH I site (sequence No. 12) inserted on the side coding for C terminus were synthesized according to sequence information (Scott Argraves, W. et al., J. Cell Biol., 105, 1183-1190 (1987)).
5' -GCGGAAAAGATGAATTTACAAC-3 ' (Sequence No. 11)
5' -GTGGGATCCTCTGGACCAGTGGGACAC-3' (Sequence No. 12)

The template cDNA, primers, dNTPs and Taq polymerase were mixed in a PCR buffer, and treated by a thermal cycler at 94°C for 1 minute for DNA denaturation, at 57°C for 2 minutes for annealing the primers and at 72°C for 3 minutes for elongating the primers. This treatment was performed 30 cycles. The amplified DNA was blunted the termini by T4DNA polymerase treatment, and digested by restriction enzyme BamH I. Then, the DNA fragment was purified. Subsequently, the DNA fragment obtained in PCR before was subcloned at the Sma I and BamH I sites of pBluescriptKS(+). A small DNA fragment purified by digesting it by restrict ion enzymes EcoR I and BamH I was inserted into a large DNA fragment of IgG₁SRα treated by restriction enzymes EcoR I and BamH I, to obtain a plasmid DNA. The plasmid (integrin β1·IgG₁SRα) is hereinafter called integrin β1·IgG heavy chain chimeric protein expression vector.

### (4) Transfection of α4·IgG heavy chain chimeric protein expression vector and integrin β1·IgG heavy chain chimeric protein expression vector into animal cells, and their expression

Integrin β1·IgG₁SRα as integrin β1·IgG heavy chain chimeric protein expression vector and pSV2dhfr (BRL) were mixed at a ratio of 10 : 1, and the mixture and lipofectin reagent (GIBCO BRL) were gently mixed and allowed to stand at room temperature for 15 minutes. The mixture was added dropwise to dihydrofolic acid reductase deficient CHO cells (ATCC CRL 9096). After 18 hours of dropwise addition, the mixture was cultured in a medium (10%FBS (GIBCO), nucleic acid-containing αMEM medium (GIBCO BRL)) for about 2 days, and the cells were dispersed by trypsin-EDTA treatment. The cells were suspended in a first selective medium (10% FBS-containing nucleic acid-free αMEM medium (GIBCO BRL)), and the suspension was disseminated into a 96-well plate (CORNING), for selective culture for about 10 days. Then, the amount of integrin β1·IgG heavy chain chimeric protein produced in the culture supernatant was determined according to the ELISA method (described later), and the clone showing the highest production was stabilized by cloning according to the limiting dilution method.

Then, into the stabilized integrin β1·IgG heavy chain chimeric protein producing CHO cells, the integrin α4·IgG heavy chain chimeric protein expression vector was transfected according to the lipofectin method as described before. That is, integrin α4·IgGSRα and pSV2neo (BRL) were mixed at 10 : 1, and the mixture was mixed with lipofectin reagent. Then, the mixture was added dropwise into the cells. After 18 hours of dropwise addition, the mixture was cultured in the said first selective medium for about 2 days, and the cells were dispersed by trypsin-EDTA treatment. The cells were suspended in a second selective medium (nucleic acid-free αMEM medium (GIBCO BRL) containing 10% FBS (GIBCO) and 1 mg/ml neomycin (GIBCO)), and on a 96-well plate (CORNING), resistant cells were selectively cultured for about 10 days. The amount of integrin α4·IgG heavy chain chimeric protein and the amount of integrin β1·IgG heavy chain chimeric protein produced in the culture supernatant were determined according to the ELISA method (described later), and a clone which produced both the chimeric proteins by almost the same amounts was picked up. The clone was cloned twice according to the limiting dilution method, to be stabilized as a clone capable of producing α4·IgG heavy chain-β1·IgG heavy chain chimeric protein heterodimer complex.

### (5) Determination of produced integrin α4·IgG heavy chain chimeric protein and integrin β1·IgG heavy chain chimeric protein by the ELISA method

Fifty microliter per well of anti-human integrin α4 antibody (Becton & Dickinson, Clone L25.3) or anti-human integrin β1 antibody (Coulter, Clone 4B4) (2µg/ml each) was put into a 96-well immunoplate (NUNC) by 50 µl/well, and allowed to stand at 4°C for 16 hours. Then, each well was washed by Dulbecco' s phosphate buffered saline (Nissui Seiyaku, not containing Ca or Mg ions, hereinafter called PBS(-)) twice, and non-specific reaction was blocked by PBS(-) containing 25% Block Ace (Snow Brand Milk Products Co., Ltd.). After blocking, the culture supernatant of CHO cells grown in selective medium was properly diluted, and reacted with the coated antibody at room temperature for 1 hour. After the reaction, the surface of the plate was washed with 0.02% Tween-containing PBS(-) (hereinafter called T-PBS) twice. It was then caused to react with biotinated anti-human IgG antibody (Vector) for 1 hour, and the reaction mixture was washed with T-PBS twice, and in succession caused to react with avidin-horseradish peroxidase (Sigma) for 1 hour. The reaction mixture was washed with PBS(-) twice. The PBS(-) was perfectly aspirated, and orthophenylenediamine was used as a substrate for color development. The absorbance at 490 nm were measured using a microplate reader (Bio-rad NOVAPATH), and the clone showing a high absorbance value was selected.

### (6) Preparation and purification of α4·IgG heavy chain-β1·IgG heavy chain chimeric protein heterodimer complex

The CHO cells highly capable of producing the α4·IgG heavy chain-β1·IgG heavy chain chimeric protein heterodimer complex were cultured in nucleic acid-free αMEM medium containing 5% FBS (Ultra-low IgG grade, GIBCO) (hereinafter called αMEM(-) medium, GIBCO BRL) for one day, to reach semiconfluent, and they were cultured in αMEM(-) medium containing 1% FBS (Ultra-low IgG grade) for 3 days, and the culture supernatant was collected. It was concentrated to 1/10 volume by ultrafiltration using Prep-scale (Millipore) , and 1M Hepes solution (pH 8.0) was added to achieve a final concentration of 5 mM, for preparing a starting solution for further purification.

FMP activated Cellulofine (Seikagaku Kogyo) was equilibrated by a coupling buffer (50 mM Na₂CO₃-NaHCO₃ pH 8.5), and a peptide showing sequence No. 13 (hereinafter called CS-1 peptide) synthesized by a peptide synthesizer was added. The mixture was inverted and mixed at 4°C for 16 hours.
Cys Leu His Gly Pro Glu Ile Leu Asp Val Pro Ser Thr (Sequence No. 13)

After completion of mixing, the mixture was washed with the coupling buffer, and a blocking buffer (0.1 mM monoethanolamine, 50 mM Tris-HCl, pH 8.0) was added. The mixture was inverted and mixed further at room temperature for 6 hours. Then, the mixture was sufficiently washed with TBS solution (150 mM NaCl, 20 mM Tris-HCl, 1 mM MnCl₂, pH 7.5), to prepare CS-1 peptide bound Cellulofine column. To the column, the starting solution for further purification was applied and all owed to stand at room temperature for 3 hours, and washed with 10 times the column volume of a washing buffer (1M NaCl, 0.1% Triton, 20 mM Tris-HCl, 1 mM MnCl₂, pH 7.5) and the same volume of the TBS solution. After completion of washing, an elution buffer (10 mM EDTA, 150 mM NaCl, 20 mM Tris-HCl, pH 7. 5) was used, to elute the proteins bound to the CS-1 column.

The eluted proteins were subjected to SDS-PAGE under non-reducing condition or reducing condition using 6.0 or 7.0% acrylamide gel, and the gel was stained with Coomassie-blue. As a result, under non-reducing condition, two bands considered to be attributable to the α4·IgG heavy chain-β1·IgG heavy chain chimeric protein heterodimer complex and its polymer were observed. Under reducing condition, two bands (170 kDa and 135 kDa) considered to be attributable to the integrin α4·IgG heavy chain chimeric protein and the integrin β1·IgG heavy chain chimeric protein and two bands (80 kDa and 90 kDa) considered to be attributable to the intramolecular cleavage of the integrin α4·IgG heavy chain chimeric protein (Hemler, M. E. et al., J. Biol. Chem., 262, 11478-11485 (1987)) were observed. These results suggest that the eluted proteins have a molecular structure considered to be α4·IgG heavy chain-β1·IgG heavy chain chimeric protein heterodimer complex, and that the molecules constituting the heterodimer are linked by a disulfide bond between the IgG heavy chains.

### (7) Identification of α4·IgG heavy chain-β1·IgG heavy chain chimeric protein heterodimer complex by immunoprecipitation

The basic method conformed to a published book ("Antibodies", Harlow, E. et al., (1988), Cold Spring Harbor Lab. Press, New York). That is, the eluted proteins of (6) considered to be α4·IgG heavy chain-β1·IgG heavy chain chimeric protein heterodimer complex were ¹²⁵I-labeled using the lactoperoxidase method. Then, Affigel-10 (Bio-rad) was washed with 0. 1 M Hepes solution (pH 8.0), and normal murine IgG, anti-human integrin α4 antibody (clone 11C2B) and anti-human integrin β1 antibody (clone 4B4) were added. Reaction was effected at 4°C for 16 hours to cause covalent bonding, to prepare normal murine IgG beads and the respective antibody beads. Then, the ¹²⁵I labeled α4·IgG heavy chain-β1·IgG heavy chain chimeric protein heterodimer complex and normal murine IgG beads were inverted and mixed at 4°C for 4 hours for preclearing, and the mixture and the antibody beads were inverted and mixed at 4°C for 16 hours. After completion of mixing, the beads were washed with a washing buffer (200 mM Tris-HCl, 0.5 M NaCl, 0.1% NP-40, 1 mM MgCl₂ or 10 mM EDTA, pH 8.0) three times. After completion of washing, a sample buffer for electrophoresis was added to the beads for treatment at 100°C for 5 minutes, and the mixture was centrifuged. The supernatant solution was analyzed by electrophoresis under reducing condition. After completion of electrophoresis, the gel was dried by a gel dryer, and the proteins were detected by autoradiography.

As a result of immunoprecipitation in the presence of 1 mM MgCl₂, from the beads of both the anti-human integrin α4 antibody and the anti-human integrin β1 antibody, the same precipitation patterns expected from the structure of α4·IgG heavy chain-β1·IgG heavy chain chimeric protein heterodimer complex were obtained. Furthermore, the pattern of immunoprecipitation by using the anti-integrin β1 antibody beads in the presence of 10 mM EDTA was similar to that in the presence of 1 mM MgCl₂. Thus, it was clarified that the proteins obtained in (6) were α4·IgG heavy chain-β1·IgG heavy chain chimeric protein heterodimer complex, and that the association did not depend on cations. This result as well as the result of (4) strongly suggests that the association of both the proteins is stable through a disulfide bond existing IgG heavy chains.

### [Example 4]

### Binding of α4·IgG heavy chain-β1·IgG heavy chain chimeric protein heterodimer complex (chimeric protein of VLA-4 and IgG heavy chain) to a ligand:

The capability of α4·IgG heavy chain-β1·IgG heavy chain chimeric protein heterodimer complex to be bound to the peptide fragment (sequence No. 13) of fibronectin as a ligand of integrin α4β1 (VLA-4) was examined.

At first, according to the said report (Humphries, M. J. et al., J. Biol. Chem., 262, 6886-6892 (1987)), the peptide fragment of sequence No. 13 (CS-1 peptide) was bound to rabbit IgG (Sigma), to prepare CS-1-IgG. The CS-1-IgG was diluted by PBS(-), and supplied in a 96-well immunoplate (NUNC) by 100 µl/well, and allowed to stand at 4°C for 16 hours, to be formed as a solid phase on the plate.

After completion of standing, the surface of the plate was washed with PBS(-) twice and treated with 1% BSA(heat-denatured at 80°C for 10 minutes)-PBS solution (300 µl/well) at 4°C for 3 hours to block the nonspecific reaction. Then, the solid phase CS-1-IgG and the CHO culture supernatant (100 µl) containing α4·IgG heavy chain-β1·IgG heavy chain chimeric protein heterodimer complex were reacted with each other at 30°C for 3 hours. The non-bound α4·IgG heavy chain-β1·IgG heavy chain chimeric protein heterodimer complex was removed by washing with 0.1% BSA-containing TBS buffer (150 mM NaCl, 25 mM Tris-HCl, 1 mM MnCl₂, pH 7.4) twice, and the bound α4·IgG heavy chain-β1·IgG heavy chain chimeric protein heterodimer complex was detected by biotin labeled anti-human IgG antibody (Vector) as the primary antibody and avidin labeled horseradish peroxidase (Sigma) used as the secondary anti body. The surface of the plate was washed with the TBS buffer. Orthophenylenediamine was added as a substrate to it for color development, and the absorbance at 490 nm were measured.

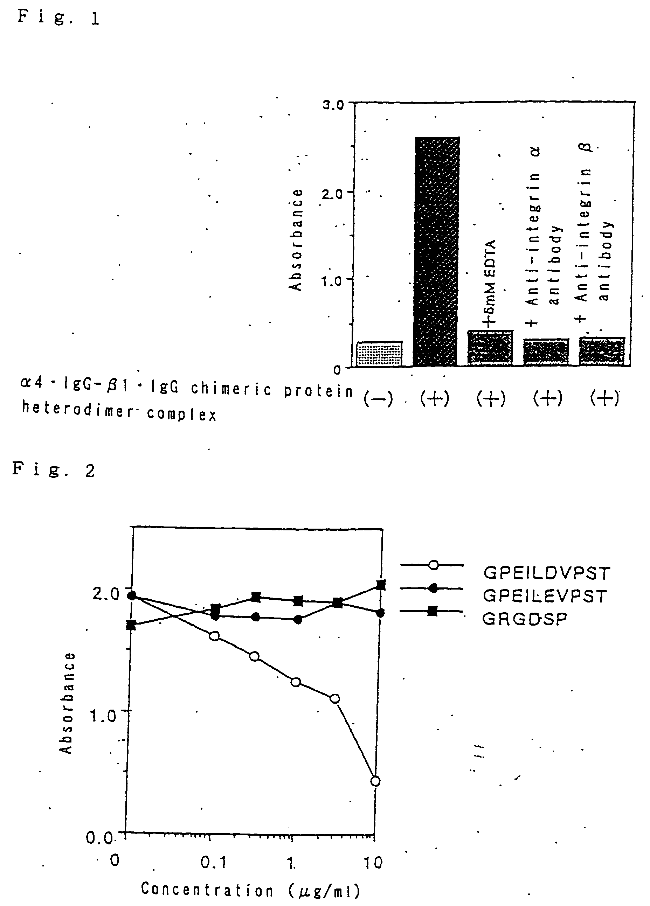

The results are shown in Fig. 1. The reaction with α4·IgG heavy chain-β1·IgG heavy chain chimeric protein heterodimer complex showed a rise in the absorbance indicating the binding to CS-1 peptide. The binding was almost perfectly inhibited by the presence of anti-integrin α4 antibody (clone L25.3), anti-integrin β1 antibody (clone 4B4) or 5 mM EDTA. Therefore, it was clarified that α4·IgG heavy chain-β1·IgG heavy chain chimeric protein heterodimer complex can be bound also to a peptide fragment of fibronectin as a ligand of integrin α4β1 (VLA-4), and that a feature of binding that it depends on cations is retained.

### [Example 5]

### Evaluation of an inhibitory peptide by using a system for determining the binding of α4·IgG heavy chain-β1·IgG heavy chain chimeric protein heterodimer complex to a ligand

In the binding determination system of Example 4, the effects of three peptides, i.e., sequence No. 14 (hereinafter called GPEILDVPST), 15 (hereinafter called GPEILEVPST) and 16 (hereinafter called GRGDSP) were examined.
Gly Pro Glu Ile Leu Asp Val Pro Ser Thr (Sequence No. 14)
Gly Pro Glu Ile Leu Glu Val Pro Ser Thr (Sequence No. 15)
Gly Arg Gly Asp Ser Pro (Sequence No. 16)

The all peptides were synthesized by a peptide synthesizer. The peptide and 100 µl of CHO cultured supernatant solution containing α4·IgG heavy chain-β1·IgG heavy chain chimeric protein heterodimer complex were mixed at room temperature for 20 minutes, and the binding to CS-1-IgG was determined according to the method in Example 4. The results are shown in Fig. 2. GPEILDVPST showed concentration-dependent inhibitory activity in a range of 0.1 to 10 µg/ml, but GPEILEVPST and GRGDSP did not show any inhibition of binding. These results show that the binding assay system in Example 4 allows to detect the inhibitory effect of the peptide (sequence No. 14) of VLA-4 bound epitope on fibronectin, i.e., the effect of VLA-4 inhibitor to be specifically detected.

### [Example 6]

### Evaluation of low molecular weight inhibitory compounds using a binding assay system between α4·IgG heavy chain-β1·IgG heavy chain chimeric protein heterodimer complex (chimeric protein of VLA-4 and IgG heavy chain) and a ligand:

Reagents and the compounds stated in literature were picked up at random, adjusted to a final concentration of 50 µg/ml, and added to the binding assay system of Example 4. Compounds showing inhibitory activity were obtained. Of the obtained compounds, the two compounds of dihydrochloric acid (Sigma) and 7-hydroxycoumarin (Aldrich, Arun. K Gas Gputa. et al., J. Chem. Soc. (C), 29-33 (1969)) were as shown in Table 2 in binding inhibitory activity. These VLA-4 inhibitory compounds may also be used for treating hepatitis like those obtained in Examples 1 and 2.

**Table 2**

| Name of compound | Concentration (µg/ml) | Inhibition rate (%) |
|---|---|---|
| Dehydrocholic acid | 50 | 39.5 |
| 7-Hydroxycoumarin | 50 | 87.5 |

### Industrial Applicability

In the present invention, it was found that integrin inhibitors, particularly VLA-4 inhibitors can be used as therapeutic agents for hepatitis.

## Claims

1. A therapeutic agent for hepatitis, comprising an integrin inhibitor as an active ingredient.

2. A therapeutic agent for hepatitis, according to claim 1, wherein the integrin inhibitor is at least one selected from a group consisting of antibodies, proteins other than antibodies, polypeptides, peptides, nucleic acids, saccharides, their derivatives and low molecular weight compounds, respectively capable of being bound to an integrin.

3. A therapeutic agent for hepatitis, according to claim 1 or 2, wherein the integrin is an integrin comprising one α chain selected from α1, α2, α3, α4, α5, α6, α7, α8, α9, αv, αL, αM, αX, α 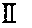 b and αE ,and one β chain selected from β1, β2, β3, β4, β5, β6, β7 and β8.

4. A therapeutic agent for hepatitis, according to claim 1 or 2, wherein the integrin is a VLA.

5. A therapeutic agent for hepatitis, according to claim 1 or 2, wherein the integrin is an integrin with α4 as the α chain.

6. A therapeutic agent for hepatitis, according to claim 4 wherein the VLA is a VLA-4.

7. A therapeutic method for hepatitis, comprising the step of administering an integrin inhibitor to a patient of hepatitis.

8. A therapeutic method for hepatitis, according to claim 7, wherein the integrin inhibitor is at least one selected from a group consisting of antibodies, proteins other than antibodies, polypeptides, peptides, nucleic acids, saccharides, their derivatives and low molecular weight compounds, respectively capable of being bound to an integrin.

9. A therapeutic method for hepatitis, according to claim 7 or 8, wherein the integrin is an integrin comprising one α chain selected from α1, α2, α3, α4, α5, α6, α7, α8, α9, αv, αL, αM, αX, α 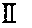 b and αE ,and one β chain selected from β1, β2, β3, β4, β5, β6, β7 and β8.

10. A therapeutic method for hepatitis, according to claim 7 or 8, wherein the integrin is a VLA.

11. A therapeutic method for hepatitis, according to claim 7 or 8, wherein the integrin is an integrin with α4 as the α chain.

12. A therapeutic method for hepatitis, according to claim 10, wherein the VLA is a VLA-4.
